# EUROPÄISCHE PATENTSCHRIFT

(11) **EP 3 165 239 B1**
(45) Veröffentlichungstag und Bekanntmachung des Hinweises auf die Patenterteilung: **04.03.2020**
(21) Anmeldenummer: 16192568.0
(22) Anmeldetag: 06.10.2016
(51) Int. Cl.: A61L 27/36, A61L 27/50

(54) **VERFAHREN ZUR REDUKTION VON PARAVALVULÄREN LECKAGEN MIT DEZELLULARISIERTEM GEWEBE**
METHOD FOR REDUCING PARAVALVULAR LEAKS WITH DECELLULARIZED TISSUE
PROCÉDÉ DE RÉDUCTION DE FUITES PARAVALVULAIRES À L'AIDE DE TISSU DÉCELLULARISÉ

(30) Priorität: 03.11.2015 DE 102015118789
(43) Veröffentlichungstag der Anmeldung: 10.05.2017
(73) Patentinhaber: Biotronik AG, 8180 Bülach (CH)
(72) Erfinder: Rzany, Alexander, 90449 Nürnberg (DE); Erdbrügger, Wilhelm, 78467 Konstanz (DE); Lehenberger, Nina, 90765 Fürth (DE)
(74) Vertreter: Randoll, Sören

(56) Entgegenhaltungen:
- EP-A1- 2 893 905
- EP-A1- 2 926 840
- US-A1- 2013 158 658

## Beschreibung

Die vorliegende Erfindung betrifft ein Verfahren zur Aufbereitung von Gewebe, insbesondere von Perikardgewebe, insbesondere porcinem Perikardgewebe, vorzugsweise für die Verwendung als Dichtmittel einer Herzklappenprothese gegen paravalvuläre Leckagen, sowie eine Herzklappenprothese, die ein derart aufbereitetes biologisches Gewebe im getrockneten Zustand enthält.

Die Erfindung wird im Folgenden am Beispiel eines Verfahrens zur Aufbereitung von Gewebe zur Verwendung als Dichtmittel für eine Herzklappenprothese beschrieben. Die vorliegende Erfindung ist zwar zur Aufbereitung derartigen Gewebes besonders geeignet, jedoch nicht auf diese Anwendung beschränkt.

Verfahren zur Aufbereitung von biologischem Gewebe sind z.B. aus der EP 2 926 840 A1, der EP 2 832 379 A1 und der US 2001/004715 A1 bekannt.

Es gibt grundsätzlich zwei verschiedene Arten von Herzklappenprothesen: mechanische Klappen, welche künstlich, zumeist aus mit pyrolytischem Kohlenstoff beschichteten Graphit, hergestellt werden, und biologische Prothesen, häufig aus Herzbeutelgewebe, welches zumeist aus tierischen Quellen (z.B. Schwein oder Rind) stammt. Die aus dem biologischen bzw. Perikardgewebe geformte Herzklappe wird zumeist in einem Grundkörper (z.B. ein festes Kunststoffgerüst oder ein selbstexpandierender Stent) befestigt und dieser wird an der Position der natürlichen Klappe implantiert. Bei solchen Herzklappenprothesen kann es sich insbesondere um sogenannte TAVI-Herzklappenprothesen handeln (TAVI für transcatheter aortic valve implantation).

Problematisch bei derartigen Prothesen sind grundsätzlich paravalvuläre Leckagen, also Blut, das zwischen der umgebenen Gefäßwand und einer Außenseite des Implantats, vorbeiströmt.

Der vorliegenden Erfindung liegt daher die Aufgabe zugrunde, ein Verfahren zur Aufbereitung von Gewebe, insbesondere Perikardgewebe, sowie eine Herzklappenprothese derart auszugestalten, dass der oben genannten Problematik entgegen gewirkt wird.

Die gestellte Aufgabe wird verfahrensseitig durch die Merkmalskombination des Anspruches 1 und vorrichtungsseitig durch die Merkmalskombination des Anspruches 16 gelöst. Vorteilhafte Ausgestaltungen dieser Aspekte der vorliegenden Erfindung sind in den entsprechenden Unteransprüchen angegeben und werden nachfolgend beschrieben.

Für das vorgeschlagene Verfahren können grundsätzlich verschiedene Arten von Gewebe, insbesondere biologischem Gewebe, und hier vom Säugetier, einschließlich vom Menschen, verwendet werden. Dabei kann das Gewebe xenogenen oder allogenen Ursprungs sein. Bevorzugt ist zunächst nicht-humanes Gewebe. Insbesondere eignen sich solche Gewebe, die ein hohes Quellvermögen aufweisen können, nachdem sie aufbereitet und getrocknet wurden. Dabei sind die Gewebe weniger einem mechanischen Stress ausgesetzt als bei einer Verwendung als Klappenmaterial in einer Herzklappe. Somit können auch Gewebsarten in Erwägung gezogen werden, die keine ausgeprägte innere Faserstruktur aufweisen, sondern vielmehr eine intrinsische schwammartige oder verzweigte Struktur. Bevorzugt für die Verwendung als Dichtmaterial von paravalvulären Leckagen sind dabei Perikardgewebe, Mucosa und Nierengewebe, aber auch Gewebe von Lunge, Magen oder Darm. Des Weiteren sind bevorzugt Gewebe vom Schwein, Schaf, Ziege, Pferd, Krokodil, Känguru, Strauß und vom Rind. Im Folgenden wird im Speziellen auf Perikardgewebe abgestellt. Der Fachmann kann jedoch ersehen, dass auch andere Gewebsarten, wie beispielsweise oben beschrieben, verwendet werden können.

Gemäß Anspruch 1 ist ein Verfahren zur Aufbereitung von Gewebe, insbesondere von Perikardgewebe, vorzugsweise für die Verwendung als Dichtmittel einer Herzklappenprothese gegen paravalvuläre Leckagen, vorgesehen, wobei das Gewebe dezellularisiert wird mittels mindestens einem starken Dezellularisierungsmittel in einer hohen Konzentration, welches die Gewebestruktur massiv verändert und offenporiger macht, anschließend einer Vernetzung mit einer Glutaraldehyd-haltigen Lösung unterzogen wird, danach einem Form- und Strukturstabilisierungsschritt unterzogen wird, bei dem das Gewebe einer ersten Lösung ausgesetzt wird, die Glycerin enthält, und einer zweiten Lösung ausgesetzt wird, die mindestens eine Art von Polyethylenglykol enthält, wobei das Gewebe nach dem Form- und Strukturstabilisierungsschritt getrocknet wird, dadurch gekennzeichnet dass, das getrocknete Gewebe vor dem Befestigen an der Herzklappenprothese zugeschnitten und warmgepresst wird.

Bei der sogenannten Dezellularisierung des Gewebes werden vorzugsweise Zellmembranen, intrazelluläre Proteine, Zellkerne und/oder andere Zellbestandteile möglichst vollständig aus dem Gewebe entfernt, um eine möglichst reine extrazelluläre Matrix zu erhalten. Im Gewebe verbleibende Zellen und Zellbestandteile stellen insbesondere eine mögliche Ursache für eine unerwünschte Kalzifizierung des biologischen Implantatmaterials dar. Die Dezellularisierung wird dabei möglichst so schonend durchgeführt, dass die Struktur der extrazellulären Matrix und Faserproteine wie beispielsweise die Kollagenfasern oder Elastin in der extrazellulären Matrix möglichst unbeeinflusst bleiben, während andererseits alle darin enthaltenen Zellen und Zellbestandteile aus dem Gewebe entfernt werden.

Im Rahmen der erfindungsgemäßen Lösung wird abweichend von gängigen Verfahren mindestens ein starkes Dezellularisierungsmittel in einer hohen Konzentration verwendet, welches die Gewebestruktur massiv verändert und offenporiger macht. Dies verbessert überraschenderweise das Quellverhalten des erfindungsgemäß behandelten Gewebes positiv. Geeignete Dezellularisierungsmittel sind ausgesucht aus der Gruppe umfassend oder bestehend aus Natriumdodecylsulfat, Desoxycholsäure, Triton X-100 und Tween. In einer bevorzugten Ausführungsform ist das Dezellularisierungsmittel Natriumdodecylsulfat.

Nach der Dezellularisierung sind möglichst alle Zellbestandteile aus dem Gewebe entfernt und das biologische Material besteht ausschließlich aus extrazellulärer Matrix. Bei Herzbeutelgewebe wird die extrazelluläre Matrix überwiegend aus Kollagenfasern gebildet. Um ein biologisches Material mit möglichst optimalen mechanischen Eigenschaften zu erreichen und Abwehrreaktionen des empfangenden Körpers zu verhindern, werden die Faserproteine bei der besagten Vernetzung bevorzugt mittels eines geeigneten Vernetzungsmittels durch den Einbau chemischer Bindungen quervernetzt. Das Vernetzungsmittel bindet an freie Aminogruppen der Faserproteine und bildet chemisch stabile Verbindungen zwischen den Faserproteinen aus. So entsteht aus den dreidimensional angeordneten Faserproteinen ein langzeitstabiles biologisches Material, welches zudem nicht mehr als biologisches Fremdmaterial erkannt wird. Durch die dreidimensionale Vernetzung bzw. Verknüpfung der einzelnen Faserproteine über das Vernetzungsmittel werden die Stabilität und die Beanspruchbarkeit des Gewebes deutlich erhöht. Dies ist insbesondere bei einem Einsatz als Gewebe und Dichtmittel in einer Herzklappe entscheidend, wo das Gewebe einer permanenten, periodischen, mechanischen Belastung ausgesetzt ist.

Die Trocknung des Gewebes, insbesondere Perikardgewebes wird bevorzugt so ausgestaltet, dass ein langsamer und schonender Entzug des Wassers im flüssigen Zustand aus dem Gewebe gewährleistet ist. Dies gelingt vorteilhafterweise z.B. durch die kontrollierte Reduktion der Umgebungsfeuchte des Gewebes in einem Klimaschrank unter kontrollierter Einstellung der Parameter der Umgebungsatmosphäre des biologischen Gewebes.

Gemäß einer besonders bevorzugten Ausführungsform des erfindungsgemäßen Verfahrens ist vorgesehen, dass das getrocknete Gewebe an einer Herzklappenprothese als Dichtmittel oder Bestandteil eines Dichtmittels gegen paravalvuläre Leckagen befestigt wird, wobei bevorzugt das Gewebe an einen expandierbaren oder selbstexpandierenden und per Katheter implantierbaren Grundkörper des Implantats befestigt, bevorzugt vernäht, wird.

Weiterhin ist gemäß einer besonders bevorzugten Ausführungsform des erfindungsgemäßen Verfahrens vorgesehen, dass das getrocknete Gewebe an einer Außenseite der Herzklappenprothese so angeordnet bzw. festgelegt wird, dass es im implantierten Zustand an einem die Herzklappenprothese umgebenden Gewebe (z.B. Gefäßwand) anliegt und einen Zwischenraum zwischen dem Gewebe und der Herzklappenprothese bei einem Rehydrieren des Gewebes aufgrund einer Dickenzunahme des rehydrierten Perikardgewebes abdichtet.

Durch die gezielte Auswahl und Kombination von Glycerin und Polyethylenglykol für den Form- und Strukturstabilisierungsschritt gelingt überraschenderweise ein gezielter Schutz der Struktur des Gewebes, insbesondere des biologischen Gewebes. Bei der gezielten Kombination von Glycerin und Polyethylenglykol, und insbesondere Polyethylenglykolen mit unterschiedlichem Molekulargewicht, wird nicht nur eine makroskopische Formstabilität des behandelten biologischen Gewebes bei der Trocknung erreicht, sondern werden die mikroskopischen Gewebestrukturen durch die Stabilisation der Wasserstoffbrücken geschützt und erhalten. Da die Eindringtiefe von Polyethylenglykol in das biologische Gewebe vom Molekulargewicht abhängt, wird eine Stabilisationswirkung in unterschiedlichen Gewebetiefen erreicht. Zusätzlich wird durch die Kombination ein spezifischer Schutz des zu trocknenden biologischen Gewebes erreicht. Glycerin und Polyethylenglykol durchdringen das Gewebe und stabilisieren die Struktur. Polyethylenglykol lagert sich zusätzlich konzentriert an der Oberfläche des Gewebes an und schirmt dieses vor äußeren Einflüssen ab. Durch die gezielte Verwendung von Polyethylenglykol in Kombination mit Glycerin, vorzugsweise in zwei Lösungen, wird die Quellfähigkeit des behandelten Gewebes infolge Stabilisierung der mikroskopischen Gewebestrukturen erhalten. Dies befördert eine Dickenzunahme bei der Rehydrierung des trockenen, erfindungsgemäß behandelten Gewebes.

Weiterhin ist gemäß einer bevorzugten Ausführungsform des erfindungsgemäßen Verfahrens vorgesehen, dass die erste Lösung Glycerin und die zweite Lösung Polyethylenglykol enthält.

Weiterhin ist gemäß einer bevorzugten Ausführungsform des erfindungsgemäßen Verfahrens vorgesehen, dass das Gewebe vor der Trocknung in dem Form- und Strukturstabilisierungsschritt einer dritten Lösung ausgesetzt wird, die Polyethylenglykol mit einem anderen, vorzugsweise größeren, mittleren Molekulargewicht als die zweite Lösung enthält.

In dieser bevorzugten Ausführungsform des erfindungsgemäßen Verfahrens kommen die Vorteile der Erfindung besonders zum Tragen. Diese Ausführungsform basiert auf der Theorie, ohne daran gebunden zu sein, dass die Eindringtiefe von Polyethylenglykol in das biologische Gewebe vom Molekulargewicht abhängt. Dies liegt mutmaßlich an der sich mit dem Molekulargewicht ändernden Viskosität. Die Verwendung von einer zweiten Polyethylenglykol enthaltenden Lösung und einer dritten Lösung, die Polyethylenglykol mit einem anderen mittleren, insbesondere größeren, Molekulargewicht als die erste Lösung enthält, ermöglicht eine Stabilisationswirkungen in unterschiedlichen Gewebetiefen. In dieser Ausgestaltung der Erfindung gelingt es entsprechend insbesondere die mikroskopischen Gewebestrukturen zu erhalten und zu stabilisieren. Weiterhin befördert die Verwendung der dritten Lösung ebenfalls eine Dickenzunahme bei der Rehydratisierung des trockenen, erfindungsgemäß behandelten Gewebes, insbesondere Perikardgewebes.

Weiterhin ist gemäß einer bevorzugten Ausführungsform des erfindungsgemäßen Verfahrens vorgesehen, dass die zweite Lösung Polyethylenglykol mit einem mittleren Molekulargewicht zwischen 100 g/mol und 1000 g/mol, bevorzugt zwischen 100 g/mol und 290 g/mol, insbesondere 200 g/mol, enthält.

Weiterhin ist gemäß einer bevorzugten Ausführungsform des erfindungsgemäßen Verfahrens vorgesehen, dass die dritte Lösung Polyethylenglykol mit einem mittleren Molekulargewicht zwischen 200 g/mol und 6000 g/mol, bevorzugt zwischen 300 g/mol und 600 g/mol, insbesondere 400 g/mol, enthält. Dabei sollte das Molekulargewicht des Polyethylenglykols größer sein als das Molekulargewicht des Polyethylenglykols der zweiten Lösung.

Weiterhin ist gemäß einer bevorzugten Ausführungsform des erfindungsgemäßen Verfahrens vorgesehen, dass das Gewebe der ersten, zweiten und/oder der dritten Lösung für 5 Minuten bis 12 Stunden, bevorzugt 15 Minuten bis 2 Stunden, weiter bevorzugt für 30 Minuten ausgesetzt wird.

Als besonders bevorzugt hat sich eine Ausgestaltung des Verfahrens erwiesen, bei der das Gewebe, insbesondere Perikardgewebe erst mit einer ersten Glycerin enthaltenden Lösung kontaktiert wird, anschließend mit der zweiten Polyethylenglykol enthaltenden Lösung kontaktiert wird und sodann mit der dritten Polyethylenglykol enthaltenden Lösung kontaktiert wird. In dieser Ausgestaltung dringt das Glycerin der ersten Lösung erst tief in das Gewebe ein, das Polyethylenglykol der zweiten Lösung (z.B. eine Lösung enthaltend Polyethylenglykol mit einem mittleren Molekulargewicht von 200 g/mol) dringt in die oberflächennahen Bereiche ein und das Polyethylenglykol der dritten Lösung (z.B. eine Lösung enthaltend Polyethylenglykol mit einem mittleren Molekulargewicht von 400 g/mol) bewirkt eine Versiegelung der Oberfläche.

Im Rahmen dieser Anmeldung bezieht sich die Angabe %v/v auf einen Volumenprozentanteil. Wird bezüglich einer Lösung nichts anderes angegeben, so werden für die Lösungen hierin Wasser als Lösungsmittel verwendet. 100 ml Lösung mit 5%v/v Glutaraldehyd enthält entsprechend 5 ml Glutaraldehyd. Die Angabe %w/v bezieht sich im Rahmen dieser Anmeldung auf einen Gewichtsanteil. 100 ml Lösung mit 0,9%w/v Natriumchlorid enthält entsprechend 0,9 g Natriumchlorid.

Weiterhin ist gemäß einer bevorzugten Ausführungsform des erfindungsgemäßen Verfahrens vorgesehen, dass Glycerin in der ersten Lösung in einer Konzentration von 5%w/v bis 50%w/v, bevorzugt 20%w/v bis 40%w/v, vorliegt.

Weiterhin ist gemäß einer bevorzugten Ausführungsform des erfindungsgemäßen Verfahrens vorgesehen, dass Polyethylenglykol in der zweiten und/oder der dritten Lösung in einer Konzentration von 5%w/v bis 60%w/v, bevorzugt von 20%w/v bis 50%w/v, vorliegt.

Weiterhin ist gemäß einer bevorzugten Ausführungsform des erfindungsgemäßen Verfahrens vorgesehen, dass das Gewebe, insbesondere Perikardgewebe in einer wässrigen Dezellularisierungslösung, enthaltend ein Dezellularisierungsmittel wie hierin beschrieben, dezellularisiert wird. Das Dezellularisierungsmittel kann dabei als gesättigte Lösung verwendet werden. In einer weiteren Ausführungsform wird eine nicht gesättigte Dezellularisierungslösung verwendet, wobei die Dezellularisierungslösung 0,1%w/v bis 15%w/v des Dezellularisierungsmittels, vorzugsweise 0,5%w/v bis 15%w/v des Dezellularisierungsmittels, vorzugsweise Natriumdodecylsulfat, vorzugsweise in 0,9%w/v NaCl oder einer vergleichbaren isotonischen wässrigen Lösung, aufweist. In einer weiteren Ausführungsform enthält die Dezellularisierungslösung 2%w/v bis 10%w/v eines hierin beschriebenen Dezellularisierungsmittels, vorzugsweise Natriumdodecylsulfat. In einer weiteren Ausführungsform enthält die Dezellularisierungslösung 7,5%w/v bis 12,5%w/v eines hierin beschriebenen Dezellularisierungsmittels, vorzugsweise Natriumdodecylsulfat. Weiterhin ist gemäß einer bevorzugten Ausführungsform des erfindungsgemäßen Verfahrens vorgesehen, dass das Gewebe, insbesondere Perikardgewebe der Dezellularisierungslösung über einen Zeitraum von 12 Stunden bis 48 Stunden, vorzugsweise 24 Stunden unter leichter Bewegung ausgesetzt wird, vorzugsweise bei einer Temperatur im Bereich von 15°C bis 40°C, vorzugsweise 37°C.

Weiterhin ist gemäß einer bevorzugten Ausführungsform des erfindungsgemäßen Verfahrens vorgesehen, dass die Glutaraldehyd-haltige Lösung 0,04%v/v bis 2%v/v Glutaraldehyd aufweist, vorzugsweise in Dulbecco's Phosphat gepufferter Salzlösung (DPBS) ohne Ca/Mg.

Weiterhin ist gemäß einer bevorzugten Ausführungsform des erfindungsgemäßen Verfahrens vorgesehen, dass das Gewebe, insbesondere Perikardgewebe der Glutaraldehyd-haltigen Lösung 1 bis 3 Tage, vorzugsweise 2 Tage, vorzugsweise bei 2°C bis 10°C, vorzugsweise bei 4°C, ausgesetzt wird, und wobei das Gewebe, insbesondere Perikardgewebe anschließend der Glutaraldehyd-haltigen Lösung 10 bis 14 Tage, vorzugsweise 12 Tage, vorzugsweise bei Raumtemperatur, die typischer Weise bei Temperaturen von 20°C bis 25°C liegt, ausgesetzt wird, wobei bevorzugt die Glutaraldehyd-haltigen Lösung alle 1 bis 3 Tage, vorzugsweise alle 2 Tage, ausgewechselt wird.

Weiterhin ist gemäß dem erfindungsgemäßen Verfahren vorgesehen, dass das getrocknete Gewebe vor dem Befestigen an der Herzklappenprothese zugeschnitten und warmgepresst wird, bevorzugt bei einer Temperatur im Bereich von 40°C bis 70°C, vorzugsweise bei 60°C.

Gemäß einem weiteren Aspekt der vorliegenden Erfindung wird eine Herzklappenprothese offenbart, aufweisend ein erfindungsgemäß behandeltes Gewebe, insbesondere Perikardgewebe, wie hierin beschrieben, wobei die Herzklappenprothese vorzugsweise eine künstliche Herzklappe sowie ein Dichtmittel aufweist, das das Gewebe, insbesondere Perikardgewebe, aufweist oder durch dieses gebildet ist, und wobei das Gewebe, insbesondere Perikardgewebe an einen expandierbaren oder selbstexpandierenden und per Katheter implantierbaren Grundkörper der Herzklappenprothese befestigt, bevorzugt vernäht, ist.

Vorzugsweise ist das Gewebe, insbesondere Perikardgewebe bzw. das Dichtmittel an einer Außenseite der Herzklappenprothese angeordnet bzw. festgelegt, so dass es im implantierten Zustand an einem die Herzklappenprothese umgebenden Gewebe (z.B. Gefäßwand) anliegt und einen Zwischenraum zwischen dem Gewebe und der Herzklappenprothese bei einem Rehydrieren des Gewebes, insbesondere Perikardgewebes aufgrund einer Dickenzunahme des rehydrierten Perikardgewebes abdichtet.

Die Möglichkeit, trockenes biologisches Gewebe so herzustellen, dass es nach Rehydrierung eine deutliche Dickenzunahme erfährt, eröffnet die Möglichkeit ein derartiges Gewebe zur Minimierung von paravalvulären Leckagen im Inflow-Bereich von (insbesondere TAVI) Herzklappen einzusetzen.

Ein weiterer Aspekt der vorliegenden Erfindung betrifft Gewebe wie hierin beschrieben, das durch ein hierin beschriebenes Verfahren behandelt wurde. Weiterhin wird dezellularisiertes und getrocknetes Gewebe vorgeschlagen, das dadurch gekennzeichnet ist, dass das Gewebe durch Rehydrierung, bevorzugt in Wasser oder Blut, eine Dickenzunahme um mindestens 110%, weiter bevorzugt um mindestens 130%, weiter bevorzugt um mindestens 150% und weiter bevorzugt um mindestens 220% aufweist im Vergleich zum nativen unbehandelten Gewebe. Weiterhin ist das dezellularisierte und getrocknete Gewebe vor der Rehydrierung ferner bevorzugt vernetzt worden. Weiterhin ist das dezellularisierte und getrocknete Gewebe vor der Rehydrierung heißgepresst worden wie hierin beschrieben. Weiterhin ist das dezellularisierte und getrocknete Gewebe vor der Rehydrierung mit Glycerin und mindestens einem Polyethylenglykol, bevorzugt mit zwei Polyethylenglykolen unterschiedlichen Molekulargewichts wie hierin beschrieben behandelt worden. Weiterhin wird die Verwendung von solchen Geweben als Dichtmaterial für paravalvuläre Leckagen insbesondere bei einer Herzklappenprothese vorgeschlagen.

Ein großer Vorteil der erfindungsgemäßen Lösung ist die Verwendung von biologischem Gewebe, z.B. Perikardgewebe, als Dichtmaterial für paravalvuläre Leckagen, was hinsichtlich thromboembolischen Komplikationen und möglichen Biokompatibilitätsproblemen verglichen mit artifiziellen Materialien deutliche Vorteile aufweist. Die resultierenden mechanischen und biokompatiblen Eigenschaften des wie beschrieben prozessierten Gewebes sind bis auf die überraschend gefundene Dickenzunahme gegenüber herkömmlich prozessierten Geweben nicht signifikant verschieden und weisen daher keine nennenswerten Nachteile auf.

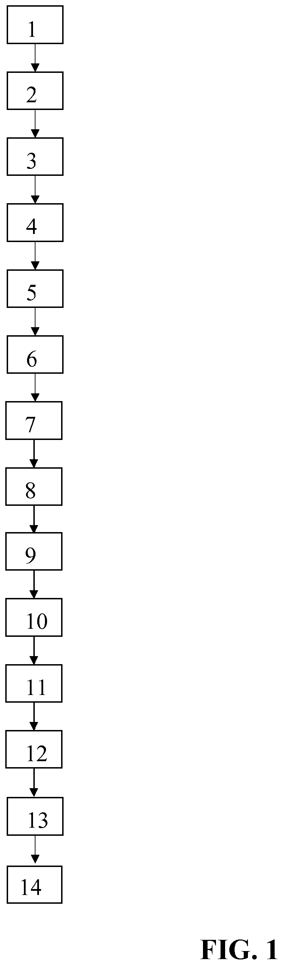

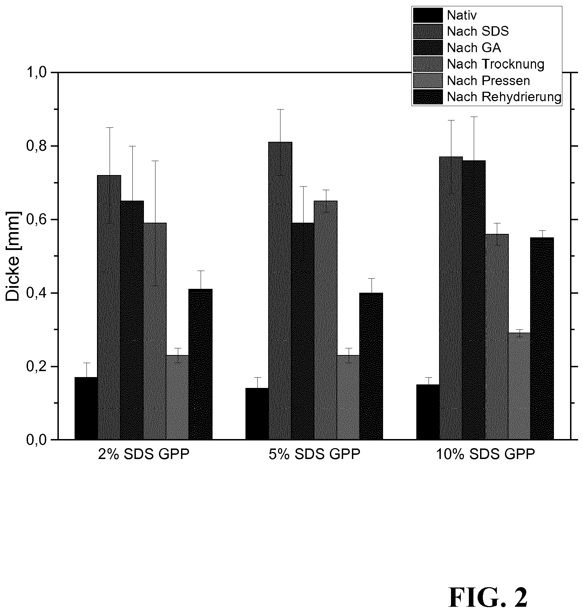

Im Folgenden soll die Erfindung anhand vom Ausführungsbeispielen (vgl. Fig. 1) und eines in der Fig. 2 dargestellten Vergleiches zwischen einem unbehandelten Perikardgewebe und einem erfindungsgemäß behandelten Perikardgewebe näher erläutert werden. Es zeigen:
- Fig. 1: ein Ablaufschema einer Ausführungsform eines erfindungsgemäßen Verfahrens; und
- Fig. 2: von links nach rechts: die Dicke von porcinem Perikard im nativen Zustand, nach anschließender Dezellularisierung in 2%w/v, 5%w/v, 10%w/v SDS in 0,9%w/v NaCl, nach anschließender Vernetzung in 0,65%v/v Glutaraldehyd in DPBS ohne Ca/Mg im Rahmen unter leichter Vorspannung, nach anschließender Stabilisation in Glycerin 30%w/v, 30 Minuten / PEG200 40%w/v, 30 Minuten / PEG400, 40%w/v, 30 Minuten (GPP, Glycerin, PEG200, PEG400) und Trocknung im Klimaschrank, nach anschließendem Warmpressen bei 60°C, 10 kg/cm2, 30 Minuten, nach anschließender Rehydrierung in 0,9%w/v NaCl, 37°C, 10 Minuten.

### Beispiel 1:

Beispiel 1 offenbart eine Ausgestaltung des erfindungsgemäßen Verfahrens zur Aufbereitung von porcinem Perikardgewebe mit anschließender Trocknung die in Figur 1 schematisch dargestellt ist.

Zunächst wird (z.B. im Schlachthof) einem Schwein ein Herzbeutel frisch entnommen und für 2 Stunden bei einer Temperatur von 4°C in einer Penicillin und/oder Streptomycin enthaltenden 0,9%w/v NaCl gelagert (1) [Schritt 1].

Im nächsten Schritt (2) werden Fett und Bindegewebe feucht (in 0,9%w/v NaCl) vom Perikardgewebe abgetrennt und das Perikardgewebe zugeschnitten.

Anschließend wird das Gewebe in 100 ml 0,9%w/v NaCl-Lösung unter leichter Bewegung gespült (3).

Das derart gewonnene Perikardgewebe wird anschließend einer Dezellularisierung und anschließender Vernetzung unterzogen.

Dabei wird das Perikardgewebe mit 100 ml 0,5%w/v bis 10%w/v SDS (Natriumdodecylsulfat) in 0,9%w/v NaCl für 24 Stunden bei 37°C unter leichter Bewegung dezellularisiert (4) und anschließend wiederholt in einer wässrigen isotonischen Lösung, vorzugsweise 0,9%w/v NaCl, unter leichter Bewegung gespült (5) (siehe auch Fig. 2).

Hiernach wird das Perikardgewebe einer Vernetzung (6) mit Glutaraldehyd unterzogen, und zwar 48 Stunden in 0,04%v/v bis 2%v/v Glutaraldehydlösung (Glutaraldehyd in gepufferter Salzlösung bei 4°C (z.B. DPBS-Lösung, Fa. Lonza; DPBS w/o Ca+/Mg+; Art.-Nr. 17-512)), wobei diese Lösung danach über 12 Tage lang bei Raumtemperatur (typisch 20°C bis 25°C) einwirkt und alle 48 Stunden gegen eine analoge, frische Lösung ausgetauscht wird).

Das derart entstandene dezellularisierte und vernetzte Perikardgewebe wird in dieser Ausgestaltung der Erfindung in drei Schritten stabilisiert, wobei das vernetzte Perikardgewebe aus Schritt (6) für 30 Minuten unter leichter Bewegung bei 37°C mit 20%w/v bis 40%w/v Glycerin in Wasser behandelt wird (7), sodann für 30 Minuten unter leichter Bewegung bei 37°C mit 20%w/v bis 50%w/v PEG200 (Polyethylenglykol 200) in Wasser behandeln wird (8), und anschließend für 30 Minuten unter leichter Bewegung bei 37°C mit 20%w/v bis 50%w/v PEG400 (Polyethylenglykol 400) in Wasser behandelt wird (9).

Hiernach wird das Perikardgewebe z.B. in einem Klimaschrank (z.B. 40°C und 10% rel. Feuchte) getrocknet (10). Wird die Trocknung unter diesen Bedingungen für 48 Stunden durchgeführt, kann die Feuchte des Gewebes von 95% auf 10% reduziert werden.

Das trockene Perikardgewebe wird zugeschnitten, um z.B. ein hierein beschriebenes Dichtmittel zu formen (11).

Weiterhin werden die getrockneten sowie zugeschnittenen Formstücke bei z.B. 60°C warmgepresst (12). Typischerweise wird das Warmpressen bei einem Druck von 2-15 MPa, bevorzugt 5-12 MPa für 5-50 Minuten, vorzugsweise 30 Minuten durchgeführt. Schritte (11) und (12) könne auch in umgekehrter Reihenfolge durchgeführt werden.

Das warmgepresste und zugeschnittene Perikardgewebe wird schließlich als Dichtmittel, wie hierin beschrieben an der Herzklappenprothese, festgelegt (13), wobei das Dichtmittel zum Abdichten paravalvulärer Leckagen konfiguriert ist.

Schließlich kann die Herzklappenprothese auf einen Katheter geladen und sterilisiert werden (14).

Wird das Perikardgewebe nunmehr am Einsatzort rehydriert, nimmt seine Dicke deutlich zu und die besagte Dichtwirkung tritt ein.

Figur 2 zeigt die absoluten Dicken in mm von porcinem Perikardgewebe in unterschiedlichen Stadien für einen exemplarischen Herstellungsprozess. Das native Gewebe mit einer Dicke kleiner 0,2 mm nimmt durch Dezellularisierung in den SDS-Lösungen (2%w/v, 5%w/v, 10%w/v) deutlich um etwa den Faktor vier in der Dicke zu. Die Vernetzung im Rahmen unter leichter Vorspannung führt zu einer leichten Abnahme der Dicke bei gleichzeitig planer Oberfläche. Die anschließende Stabilisation in Lösungen aus Glycerin/PEG200/PEG400 mit anschließender Trocknung im Klimaschrank (10% rel. Luftfeuchte) ändert die Dicke nicht signifikant. Durch Warmpressen lässt sich die Dicke des so vorbehandelten porcinen Perikards gezielt um mindestens den Faktor zwei bis drei reduzieren. Die Gewebedicke liegt nach dem Warmpressen wieder im Bereich des nativen Gewebes, insbesondere für die niedrigeren SDS-Konzentrationen. Durch abschließendes Rehydrieren in 0,9%w/v Kochsalzlösung quillt das gepresste porcine Perikard deutlich auf und die Dicke nimmt um etwa 100% zu.

Im Ausführungsbeispiel wird vorzugsweise porcines Perikardgewebe als Ausgangsmaterial verwendet, das bei TAVI-Klappen zum Einsatz kommt. Zur Abdichtung von paravalvulären Leckagen sind jedoch die mechanischen Anforderungen an das Gewebe geringer als für Klappenflügel. Wie bereits angeführt ist auch die Verwendung von anderen biologischen Geweben ohne ausgeprägte innere Faserstruktur implementierbar, welche eine intrinsische schwammartige oder verzweigte Struktur aufweisen können, z.B. porcines oder bovines Nieren-, Magen- oder Darmgewebe.

## Patentansprüche

1. Verfahren zur Aufbereitung von Gewebe für die Verwendung als Dichtmittel einer Herzklappenprothese gegen paravalvuläre Leckagen, wobei das Gewebe dezellularisiert (4) wird mittels mindestens einem starken Dezellularisierungsmittel in einer hohen Konzentration, welches die Gewebestruktur massiv verändert und offenporiger macht, anschließend einer Vernetzung (6) mit einer Glutaraldehyd-haltigen Lösung unterzogen wird, danach einem Form- und Strukturstabilisierungsschritt unterzogen wird (7, 8, 9), bei dem das Gewebe einer ersten Lösung ausgesetzt wird (7), die Glycerin enthält, und einer zweiten Lösung ausgesetzt wird (8), die Polyethylenglykol enthält, wobei das Gewebe nach dem Form- und Strukturstabilisierungsschritt getrocknet wird, **dadurch gekennzeichnet dass,** das getrocknete Gewebe vor dem Befestigen an der Herzklappenprothese zugeschnitten und warmgepresst (12) wird.

2. Verfahren nach Anspruch 1, **dadurch gekennzeichnet dass,** das Dezellularisierungsmittel ausgewählt ist aus der Gruppe umfassend oder bestehend aus Natriumdodecylsulfat, Desoxycholsäure, Triton X-100 und Tween, bevorzugt Natriumdodecylsulfat.

3. Verfahren nach Anspruch 1 oder 2, **dadurch gekennzeichnet dass,** das getrocknete Gewebe an einer Herzklappenprothese als Dichtmittel oder Bestandteil eines Dichtmittels gegen paravalvuläre Leckagen befestigt wird (13), wobei bevorzugt das Gewebe an einem expandierbaren oder selbstexpandierenden und per Katheter implantierbaren Grundkörper des Implantats befestigt wird.

4. Verfahren nach einem der Ansprüche 1 bis 3, **dadurch gekennzeichnet dass,** das getrocknete Gewebe an einer Außenseite der Herzklappenprothese angeordnet wird (13), so dass es im implantierten Zustand an einem die Herzklappenprothese umgebenden Gewebe anliegt und einen Zwischenraum zwischen dem Gewebe und der Herzklappenprothese bei einem Rehydrieren des Gewebes aufgrund einer Dickenzunahme des rehydrierten Gewebes abdichtet.

5. Verfahren nach einem der vorhergehenden Ansprüche, **dadurch gekennzeichnet dass,** das Gewebe vor der Trocknung (10) in dem Form- und Strukturstabilisierungsschritt (7, 8, 9) einer dritten Lösung ausgesetzt wird (9), die Polyethylenglykol mit einem anderen mittleren Molekulargewicht als die zweite Lösung enthält.

6. Verfahren nach Anspruch 5, **dadurch gekennzeichnet dass,** die dritte Lösung Polyethylenglykol mit einem mittleren Molekulargewicht zwischen 200 g/mol und 6000 g/mol enthält.

7. Verfahren nach einem der Ansprüche 5 oder 6, **dadurch gekennzeichnet dass,** das Gewebe der ersten, zweiten und/oder der dritten Lösung für 5 Minuten bis 12 Stunden ausgesetzt wird.

8. Verfahren nach einem der Ansprüche 5 bis 7, **dadurch gekennzeichnet dass** Polyethylenglykol in der zweiten und/oder der dritten Lösung in einer Konzentration von 5% w/v bis 60% w/v vorliegt.

9. Verfahren nach einem der vorangegangenen Ansprüche, **dadurch gekennzeichnet dass** Glycerin in der ersten Lösung in einer Konzentration von 5% w/v bis 50% w/v vorliegt.

10. Verfahren nach einem der vorangegangenen Ansprüche, **dadurch gekennzeichnet dass,** die zweite Lösung Polyethylenglykol mit einem mittleren Molekulargewicht zwischen 100 g/mol und 1000 g/mol, insbesondere 200 g/mol, enthält.

11. Verfahren nach einem der vorangegangen Ansprüche, **dadurch gekennzeichnet dass,** das Gewebe in einer wässrigen Dezellularisierungslösung, die ein Dezellularisierungsmittel enthält, dezellularisiert wird (4), wobei die Dezellularisierungslösung vorzugsweise 0,1% w/v bis 15% w/v, vorzugsweise 0,5% w/v bis 15% w/v des Dezellularisierungsmittels, vorzugsweise in 0,9% w/v NaCl oder einer vergleichbaren isotonischen wässrigen Lösung, aufweist.

12. Verfahren nach Anspruch 11, **dadurch gekennzeichnet dass,** das Gewebe der Dezellularisierungslösung über einen Zeitraum von 12 Stunden bis 48 Stunden ausgesetzt wird (4), vorzugsweise bei einer Temperatur im Bereich von 15°C bis 40°C.

13. Verfahren nach einem der vorangegangenen Ansprüche, **dadurch gekennzeichnet dass,** das Gewebe xenogenen oder allogenen Ursprungs ist, vorzugsweise ausgewählt ist aus der Gruppe umfassend oder bestehend aus Perikardgewebe, Mucosa, Nierengewebe, sowie Gewebe von Lunge, Magen oder Darm.

14. Verfahren nach einem der vorangegangenen Ansprüche, **dadurch gekennzeichnet dass,** das getrocknete Gewebe bei einer Temperatur im Bereich von 40°C bis 70°C warmgepresst (12) wird.

15. Dezellularisiertes, Gewebe das einem Verfahren gemäß einem der Ansprüche 1 bis 14 unterzogen wurde, **dadurch gekennzeichnet dass,** das Gewebe durch Rehydrierung eine Dickenzunahme um mindestens 110% aufweist im Vergleich zum nativen unbehandelten Gewebe.

16. Herzklappenprothese, aufweisend ein Gewebe nach Anspruch 15 oder ein Gewebe, welches einem Verfahren gemäß einem der Ansprüche 1 bis 14 unterzogen wurde, wobei die Herzklappenprothese ein Dichtmittel aufweist, welches das Gewebe aufweist oder durch dieses gebildet ist, wobei das Gewebe an einen expandierbaren oder selbstexpandierenden und per Katheter implantierbaren Grundkörper der Herzklappenprothese befestigt ist, und zwar vorzugsweise an einer Außenseite der Herzklappenprothese, so dass es im implantierten Zustand an einem die Herzklappenprothese umgebenden Gewebe anliegt und einen Zwischenraum zwischen dem Gewebe und der Herzklappenprothese bei einem Rehydrieren des Gewebes aufgrund einer Dickenzunahme des rehydrierten Gewebes abdichtet.

## Claims

1. A method for preparing tissue for use as a sealing means for sealing a heart valve prosthesis with respect to paravalvular leaks, wherein the tissue is decellularized (4) by means of at least one strong decellularization agent in a high concentration, which significantly changes the tissue structure and makes it open-pored, then is subjected to a cross-linking (6) with a glutaraldehyde-containing solution, then is subjected to a shape- and structure-stabilising step (7, 8, 9), in which the tissue is exposed to a first solution (7) containing glycerol and is exposed to a second solution (8) containing polyethylene glycol, wherein the tissue is dried after the shape- and structure-stabilising step, **characterized in that** the dried tissue, prior to being fastened to the heart valve prosthesis, is cut to size and hot-pressed (12).

2. The method according to claim 1, **characterized in that** the decellularization agent is selected from the group comprising or consisting of sodium dodecyl sulphate, deoxycholic acid, Triton X-100, and Tween, preferably sodium dodecyl sulphate.

3. The method according to claim 1 or 2, **characterized in that** the dried tissue is fastened (13) to a heart valve prosthesis as sealing means or part of a sealing means for sealing against paravalvular leaks, wherein the tissue is preferably fastened to an expandable or self-expanding main body of the implant, which can be implanted by catheter.

4. The method according to any one of claims 1 to 3, **characterized in that** the dried tissue is arranged on an outer side of the heart valve prosthesis (13) so that, in the implanted state, it bears against tissue surrounding the heart valve prosthesis and, as the tissue is rehydrated, seals a gap between the tissue and the heart valve prosthesis on account of an increase in the thickness of the rehydrated tissue.

5. The method according to any one of the preceding claims, **characterized in that** the tissue is exposed to a third solution (9) containing polyethylene glycol having a mean molecular weight different from the second solution, prior to the drying (10) in the shape- and structure-stabilising step (7, 8, 9).

6. The method according to claim 5, **characterized in that** the third solution contains polyethylene glycol having a mean molecular weight between 200 g/mol and 6,000 g/mol.

7. The method according to either one of claims 5 or 6, **characterized in that** the tissue is exposed to the first, second and/or the third solution for 5 minutes to 12 hours.

8. The method according to any one of claims 5 to 7, **characterized in that** polyethylene glycol is present in the second and/or the third solution in a concentration of from 5%w/v to 60%w/v.

9. The method according to any one of the preceding claims, **characterized in that** the glycerol is present in the first solution in a concentration of from 5%w/v to 50%w/v.

10. The method according to any one of the preceding claims, **characterized in that** the second solution contains polyethylene glycol having a mean molecular weight between 100 g/mol and 1,000 g/mol, in particular 200 g/mol.

11. The method according to any one of the preceding claims, **characterized in that** the tissue is decellularized (4) in an aqueous decellularization solution containing a decellularization agent, wherein the decellularization solution preferably comprises 0.1%w/v to 15%w/v, preferably 0.5%w/v to 15%w/v of the decellularization agent, preferably in 0.9%w/v NaCl or a comparable isotonic aqueous solution.

12. The method according to claim 11, **characterized in that** the tissue is exposed to the decellularization solution over a period of from 12 hours to 48 hours (4), preferably at a temperature in the range of from 15°C to 40°C.

13. The method according to any one of the preceding claims, **characterized in that** the tissue is of xenogeneic or allogeneic origin, preferably selected from the group comprising or consisting of pericardial tissue, mucosa, kidney tissue, and tissue from the lung, stomach or intestine.

14. The method according to any one of the preceding claims, **characterized in that** the dried tissue is hot-pressed (12) at a temperature in the range of from 40°C to 70°C.

15. A decellularized tissue that has been subjected to a method according to any one of claims 1 to 14, **characterized in that** the tissue, by means of rehydration, has an increase in thickness by at least 110% compared to the native untreated tissue.

16. A heart valve prosthesis, comprising a tissue according to claim 15 or a tissue which has been subjected to a method according to any one of claims 1 to 14, wherein the heart valve prosthesis comprises a sealing means which comprises the tissue or which is formed thereby, wherein the tissue is fastened to an expandable or self-expanding main body of the heart valve prosthesis, which can be implanted by catheter, more specifically is preferably fastened to an outer side of the heart valve prosthesis, such that, in the implanted state, it bears against a tissue surrounding the heart valve prosthesis and, as the tissue is rehydrated, seals off a gap between the tissue and the heart valve prosthesis on account of an increase in the thickness of the rehydrated tissue.

## Revendications

1. Procédé de préparation d'un tissu pour l'emploi en tant que produit d'étanchéité d'une prothèse de valvule cardiaque contre des fuites paravalvulaires, où le tissu est décellularisé (4) au moyen d'au moins un produit de décellularisation puissant en concentration élevée, lequel modifie de manière considérable la structure du tissu et crée des pores ouvertes, est ensuite soumis à une réticulation (6) avec une solution contenant du glutaraldéhyde, puis est soumis à une étape de façonnage et de stabilisation de la structure (7, 8, 9), dans laquelle le tissu est soumis (7) à une première solution qui contient de la glycérine, et est soumis (8) à une deuxième solution qui contient du polyéthylène glycol, où le tissu est séché après l'étape de façonnage et de stabilisation de la structure, **caractérisé en ce que** le tissu séché est découpé et pressé à chaud (12) avant la fixation sur la prothèse de valvule cardiaque.

2. Procédé selon la revendication 1, **caractérisé en ce que** le produit de décellularisation est choisi dans le groupe comprenant ou étant constitué de dodécyl sulfate de sodium, d'acide désoxycholique, de Triton X-100 ou de Tween, de préférence de dodécyl sulfate de sodium.

3. Procédé selon la revendication 1 ou la revendication 2, **caractérisé en ce que** le tissu séché est fixé (13) sur une prothèse de valvule cardiaque en tant que produit d'étanchéité ou composant d'un produit d'étanchéité contre des fuites paravasculaires, où, de préférence, le tissu est fixé sur un corps de base de l'implant pouvant être expansé ou s'auto expansant et implantable par cathéter.

4. Procédé selon l'une des revendications 1 à 3, **caractérisé en ce que** le tissu séché est placé (13) sur un côté extérieur de la prothèse de valvule cardiaque de sorte qu'il se plaque à l'état implanté contre un tissu entourant la prothèse de valvule cardiaque et rend étanche un espace intermédiaire entre le tissu et la prothèse de valvule cardiaque lors d'une réhydratation du tissu en raison d'une augmentation d'épaisseur du tissu réhydraté.

5. Procédé selon l'une des revendications précédentes, **caractérisé en ce que** le tissu est soumis (9) à une troisième solution qui contient du polyéthylène glycol avec un poids moléculaire moyen différent de la deuxième solution avant le séchage (10) dans l'étape de façonnage et de stabilisation de la structure (7, 8, 9).

6. Procédé selon la revendication 5, **caractérisé en ce que** la troisième solution contient du polyéthylène glycol avec un poids moléculaire moyen entre 200 g/mol et 6000 g/mol.

7. Procédé selon l'une des revendications 5 ou 6, **caractérisé en ce que** le tissu est soumis à la première, à la deuxième et/ou à la troisième solution pendant 5 minutes à 12 heures.

8. Procédé selon l'une des revendications 5 à 7, **caractérisé en ce que** le polyéthylène glycol est présent dans une concentration de 5 % en poids/volume à 60 % en poids/volume dans la deuxième et/ou la troisième solution.

9. Procédé selon l'une des revendications précédentes, **caractérisé en ce que** de la glycérine est présente dans la première solution dans une concentration de 5 % en poids/volume à 50 % en poids/volume.

10. Procédé selon l'une des revendications précédentes, **caractérisé en ce que** la deuxième solution contient du polyéthylène glycol avec un poids moléculaire moyen entre 100 g/mol et 1000 g/mol, notamment 200 g/mol.

11. Procédé selon l'une des revendications précédentes, **caractérisé en ce que** le tissu est décellularisé (4) dans une solution de décellularisation aqueuse qui contient un produit de décellularisation, où la solution de décellularisation présente de préférence 0,1 % en poids/volume à 15 % en poids/volume, de préférence 0,5 % en poids/volume à 15 % en poids/volume de produit de décellularisation, de préférence dans une solution aqueuse isotonique à 0,9 % en poids/volume de NaCl ou similaire.

12. Procédé selon la revendication 11, **caractérisé en ce que** le tissu est soumis (4) à la solution de décellularisation pendant une durée de 12 heures à 48 heures, de préférence, à une température dans la plage de 15 °C à 40 °C.

13. Procédé selon l'une des revendications précédentes, **caractérisé en ce que** le tissu est d'origine xénogène ou allogène, est choisi de préférence dans le groupe comprenant ou constitué de tissu péricardique, de muqueuse, de tissu rénal, ainsi que de tissu de poumon, d'estomac ou d'intestin.

14. Procédé selon l'une des revendications précédentes, **caractérisé en ce que** le tissu séché est pressé à chaud (12) à une température dans la plage de 40 °C à 70 °C.

15. Tissu décellularisé qui a été soumis à un procédé selon l'une des revendications 1 à 14, **caractérisé en ce que** le tissu présente une augmentation d'épaisseur d'au moins 110 % par la réhydratation comparativement à un tissu d'origine non traité.

16. Prothèse de valvule cardiaque, présentant un tissu selon la revendication 15 ou un tissu, lequel a été soumis à un procédé selon l'une des revendications 1 à 14, où la prothèse de valvule cardiaque présente un produit d'étanchéité, lequel présente le tissu ou en est formé, où le tissu est fixé à un corps de base de la prothèse de valvule cardiaque pouvant être expansé ou s'auto expansant et implantable par cathéter, et en l'occurrence, de préférence, sur un côté extérieur de la prothèse de valvule cardiaque, de sorte qu'il se plaque à l'état implanté contre un tissu entourant la prothèse de valvule cardiaque et rend étanche un espace intermédiaire entre le tissu et la prothèse de valvule cardiaque lors d'une réhydratation du tissu en raison d'une augmentation d'épaisseur du tissu réhydraté.
